# EUROPÄISCHE PATENTSCHRIFT

(11) **EP 2 626 007 B1**
(45) Veröffentlichungstag und Bekanntmachung des Hinweises auf die Patenterteilung: **19.02.2014**
(21) Anmeldenummer: 12164853.9
(22) Anmeldetag: 20.04.2012
(51) Int. Cl.: A61B 5/15, A61M 5/32

(54) **Sicherheitsvorrichtung für eine Kanüle**
Safety device for a cannula
Dispositif de sécurité pour une canule

(30) Priorität: 07.02.2012 DE 102012100971
(43) Veröffentlichungstag der Anmeldung: 14.08.2013
(73) Patentinhaber: KD Medical GmbH Hospital Products, 10117 Berlin (DE)
(72) Erfinder: Brietzke, Ursula, 10117 Berlin (DE)
(74) Vertreter: Kilchert, Jochen

(56) Entgegenhaltungen:
- EP-A1- 0 827 754
- DE-T2- 69 529 140
- US-A- 5 599 318
- US-B1- 6 413 243

## Beschreibung

Die Erfindung bezieht sich auf eine Sicherheitsvorrichtung für eine Kanüle mit den Merkmalen des Oberbegriffs des Anspruchs 1. Eine Kanüle dieser Gattung ist beispielsweise aus EP 1 433 419 A1 bekannt.

Bei der Handhabung von Kanülen durch medizinisches Personal stellen Nadelstichverletzungen durch Kanülenspitzen, die mit Patientenblut oder Körperflüssigkeiten verunreinigt sein, ein gravierendes Problem dar. Kanülen bestehen üblicherweise aus einer Nadelröhre, einer Nadelbasis, einem Konnektor und einer Schutzhülle. Die Schutzhülle wird dazu verwendet, um die Nadelröhre zu schützen und Nadelstichverletzungen zu vermeiden. Dazu ist die Schutzhülle an der Außenseite der Nadelbasis befestigt. Vor dem Gebrauch der Kanüle deckt die Schutzhülle die Kanüle am Spritzenansatz durch ein Loch an der Nadelbasis ab. Für den Gebrauch der Kanüle wird die Schutzhülle abgezogen, so dass die Kanüle für das Ansaugen oder Injizieren von Arzneimitteln einsatzbereit ist. In den meisten Fällen werden die Schutzhüllen nach Beendigung der Injektion entsorgt. Durch die nach der Injektion frei zugängliche Kanülenspitze besteht dann ein besonders großes Risiko von Nadelstichverletzungen. Dieses Risiko setzt das medizinische Personal nicht nur schweren psychischen Belastungen aus, sondern stellt auch ein besonderes Problem für das mit der Entsorgung von medizinischem Müll beauftragte Personal dar.

Bei der aus der eingangs genannten EP 1 433 419 A1 bekannten Kanüle ist ein automatisch verschließbarer Schutzmechanismus vorgesehen, bei dem die Schutzhülle nach Beendigung der Injektion bzw. Punktion durch eine Federkraft verschwenkt wird und die Kanülenspitze schützt. Dazu ist am proximalen Ende der Schutzhülle ein Rastarm vorgesehen, der in zwei verschiedenen Positionen mit dem Kanülengehäuse verriegelbar ist. Zum Lösen des Armes wird in der Offenstellung der Schutzhülle ein Betätigungselement in axialer Richtung verschoben, das den Arm zur Seite drückt und die Rastverbindung löst. Die mit einer Federkraft beaufschlagte Schutzhülle wird über die Kanüle gedreht, bis der Arm in einer zweiten Position wieder verrastet. Der Nachteil der bekannten Kanüle besteht darin, dass für die automatische Schließfunktion viele bewegliche Bauteile erforderlich sind, wodurch nicht nur die Fehleranfälligkeit der Kanüle erhöht, sondern auch der Fertigungsaufwand beträchtlich steigt. Gerade wenn man bedenkt, dass es sich bei der Kanüle um ein Einwegprodukt handelt, sind die aufgrund des aufwändigen Aufbaus der Kanüle hohen Fertigungskosten besonders schwerwiegend. Außerdem ist es bei der bekannten Kanüle erforderlich, vor dem Gebrauch eine Schutzkappe, die zusätzlich zu der schwenkbaren Schutzhülle vorgesehen ist, von der Kanüle abzuziehen. Das medizinische Personal muss also vor der Injektion im Bereich der frei zugänglichen Kanülenspitze hantieren, wodurch das Verletzungsrisiko steigt.

Der Erfindung liegt die Aufgabe zu Grunde, eine Sicherheitskanüle anzugeben, die einfach aufgebaut ist und eine sichere Benutzung von Kanülen durch das medizinische Personal erlaubt.

Erfindungsgemäß wird diese Aufgabe durch eine Sicherheitsvorrichtung für eine Kanüle mit den Merkmalen des Anspruchs 1 gelöst. Bevorzugte Ausführungsformen der Sicherheitsvorrichtung sind in den Unteransprüchen angegeben.

Die Aufgabe wird insbesondere durch eine Sicherheitsvorrichtung für eine Kanüle gelöst, die ein Verriegelungselement und eine Schutzhülle umfasst, wobei die Schutzhülle mit einem Basisteil verbunden ist und einen Hohlraum mit einer Aufnahmeöffnung für die Kanüle aufweist. Die Schutzhülle ist in eine Offenstellung und in eine Schließstellung um eine Schwenkachse schwenkbar. In der Schließstellung ist eine im Basisteil positionierbare Kanüle im Hohlraum geschützt angeordnet. Die Schutzhülle ist in der Schließstellung durch das Verriegelungselement arretierbar. Das Verriegelungselement ist um eine parallel zur Schwenkachse verlaufende Drehachse relativ zur Schutzhülle von einer Ruhestellung in eine Raststellung drehbar. Das Verriegelungselement ist zur Erzeugung der Drehbewegung durch eine Schwenkbewegung der Schutzhülle von der Schließstellung in die Offenstellung betätigbar.

Zum Schutz des medizinischen Personals ist die Kanüle in der Schließstellung im Hohlraum angeordnet, wobei die Schutzhülle zusätzlich durch das Verriegelungselement arretierbar ist, so dass ein unbeabsichtigtes Öffnen der Schutzhülle beim Entsorgen der Kanüle verhindert wird.

Für die Arretierfunktion des Verriegelungselements ist dieses von einer Ruhestellung in eine Raststellung drehbar. Das Verriegelungselement nimmt in der Raststellung die Position relativ zur Schutzhülle ein, die für die Erfüllung der Arretierfunktion Voraussetzung ist. Die Drehung des Verriegelungselements erfolgt um eine parallel zur Schwenkachse der Schutzhülle verlaufende Drehachse. Dadurch wird erreicht, dass die Drehbewegung des Verriegelungselements und die Schwenkbewegung der Schutzhülle in einer Ebene erfolgen. Durch die Parallelanordnung der Schwenkachse und der Drehachse wird der Aufbau der Sicherheitsvorrichtung signifikant vereinfacht, weil für die Arretierfunktion lediglich zwei bewegliche Bauteile, nämlich das Verriegelungselement und die Schutzhülle erforderlich sind, wobei nicht ausgeschlossen ist, dass die Sicherheitsvorrichtung mehr als zwei bewegliche Bauteile aufweist. Außerdem erfüllt die Schutzhülle eine Doppelfunktion. Zum einen vermeidet die Schutzhülle eine Berührung der Kanülenspitze durch das medizinische Personal, wenn die Kanüle in der Schließstellung der Schutzhülle im Hohlraum angeordnet ist. Zum anderen fungiert die Schutzhülle als Betätigungselement, das die Drehbewegung des Verriegelungselements verursacht. Zur Erzeugung der Drehbewegung des Verriegelungselements ist dieses durch die Schwenkbewegung der Schutzhülle, insbesondere durch eine Schwenkbewegung der Schutzhülle von der Schließstellung in die Offenstellung betätigbar. Damit wird eine Drehung des Verriegelungselements relativ zur Schutzhülle erreicht. Die Kopplung der Schwenkbewegung der Schutzhülle mit der Drehbewegung des Verriegelungselements erhöht die Sicherheit der Handhabung der Sicherheitsvorrichtung, weil das Verriegelungselement durch die Bewegung der Schutzhülle automatisch in die Raststellung bewegt wird.

Bei einer bevorzugten Ausführungsform der Sicherheitsvorrichtung weist das Verriegelungselement einen Kipphebel auf, der durch eine Durchgangsöffnung nach außen ragt, die in einer Wandung der Schutzhülle gegenüber der Aufnahmeöffnung angeordnet ist. Dadurch wird erreicht, dass das Verriegelungselement auf der Außenseite der Schutzhülle zugänglich ist und somit manipuliert werden kann, insbesondere durch die Schutzhülle.

Bei einer besonders bevorzugten Ausführungsform ist der Kipphebel bei der Schwenkbewegung der Schutzhülle von der Schließstellung in die Offenstellung am Basisteil derart abstützbar, dass das Verriegelungselement in die Raststellung bewegbar ist. Dadurch wirkt das Basisteil mit der Schutzhülle bei der Betätigung des Verriegelungselements zusammen. Das Basisteil bildet somit eine Art Widerlager und der Kipphebel einen auf einer Kreisbahn geführten Nocken, der auf dem Widerlager aufliegt. Der Nocken bzw. Kipphebel folgt der Schwenkbewegung der Schutzhülle und liegt gleichzeitig auf dem Widerlager bzw. Basisteil auf, wodurch ein Drehmoment über den Kipphebel eingeleitet wird. Das freie Ende des Kipphebels führt dabei eine translatorische Bewegung entlang der Oberfläche des Basisteils aus. Gleichzeitig rollt das freie Ende auf der Oberfläche des Basisteils ab. Damit erfüllt das Basisteil eine Doppelfunktion, da dieses einerseits für die Positionierung der Kanüle verantwortlich ist und andererseits zusammen mit der Schutzhülle als Betätigungselement für den Kipphebel dient.

Wenn der Abstand zwischen der Drehachse des Verriegelungselements und der Schwenkachse der Schutzhülle kleiner als der Abstand zwischen der Drehachse und dem freien Ende des Kipphebels ist, wird sicher erreicht, dass das freie Ende des Kipphebels mit dem Basisteil beim Verschwenken der Schutzhülle in Anlage kommt bzw. in Anlage bleibt.

Die Relativbewegung zwischen dem Kipphebel und dem Basisteil beim Verschwenken der Schutzhülle wird dadurch verbessert, dass der Kipphebel am freien Ende eine abgerundete Form zum Gleiten auf den Basisteilen aufweist.

Bei einer weiteren bevorzugten Ausführungsform ist das Verriegelungselement zum Arretieren der sich in der Schließstellung befindlichen Schutzhülle in der Raststellung mit der Kanüle verrastbar. Diese Ausführungsform trägt zum einfachen Aufbau der Sicherheitsvorrichtung bei, da zum Arretieren der Schutzhülle keine zusätzlichen Bauteile erforderlich sind, sondern die ohnehin vorhandene Kanüle verwendet wird. Außerdem wird die Sicherheit verbessert, da die Verbindung zwischen dem Verriegelungselement und der Kanüle in dem nicht oder schwer zugänglichen Hohlraum der Schutzhülle erfolgt, wodurch ein unbeabsichtigtes Lösen der Rastverbindung zwischen dem Verriegelungselement und der Kanüle vermieden wird.

Das Verriegelungselement kann einen Rasthaken aufweisen, der eine Aufnahmemulde für die Kanüle aufweist. Die Aufnahmemulde erstreckt sich in der Schließstellung der Schutzhülle und der Raststellung des Verriegelungselements koaxial zu einer ersten Öffnung des Basisteils. Die erste Öffnung des Basisteils kann die Kanüle, insbesondere einen Kanülenansatz aufnehmen. Das Verriegelungselement kann insbesondere einen Rasthaken aufweisen, der sich im Wesentlichen seitlich erstreckt, also eine seitlich bzw. parallel zur Drehachse weisende Hakenspitze bildet. Der Rasthaken ist mit der Kanüle in Eingriff bringbar, wodurch eine besonders einfache, insbesondere einfach herzustellende Rastverbindung geschaffen wird.

Bei einer weiteren besonders bevorzugten Ausführungsform weist die Schutzhülle mindestens ein erstes Arretiermittel auf, das an einer Innenseite der Schutzhülle befestigt, insbesondere ausgeformt, ist, um das Verriegelungselement in der Raststellung zu fixieren. Dies hat den Vorteil, dass das Verriegelungselement durch die Fixierung so ausgerichtet werden kann, dass dieses sicher in die Kanüle oder in ein anderes Fixierungsbauteil eingreift. Eine Falschstellung des Verriegelungselements beim Arretieren wird dadurch vermieden.

Bei einer bevorzugten Ausführungsform ist das Verriegelungselement in der Ruhestellung durch das erste Arretiermittel lösbar fixiert. Das erste Arretiermittel dient damit sowohl zur Fixierung des Verriegelungselements in der Ruhestellung als auch in der Raststellung, wobei zum Überführen des Verriegelungselements von der Ruhestellung in die Raststellung dieses lösbar mit dem ersten Arretiermittel fixiert ist. Das erste Arretiermittel erfüllt also eine Doppelfunktion, was wiederum zu einer Reduzierung der erforderlichen Bauteile beiträgt.

Das Arretiermittel kann eine Gleitschräge aufweisen, die in proximale Richtung entlang der Innenwand ansteigt und in einer Rotationsebene des Verriegelungselements derart angeordnet ist, dass das Verriegelungselement bei der Bewegung von der Ruhestellung in die Raststellung auf der Gleitschräge gleitet. Bei der Bewegung des Verrieglungselements, insbesondere des Rasthakens entlang der Gleitschräge kann vorgesehen sein, dass der Rasthaken parallel zur Drehachse des Verriegelungselements ausgelenkt wird. Die Auslenkung erfolgt vorzugsweise in Richtung derselben Innenwand der Schutzhülle, an welcher das Arretiermittel angeordnet bzw. ausgeformt ist. Über den Anpressdruck zwischen der Gleitschräge und dem Verriegelungselement kann der Widerstand gesteuert werden, der zu überwinden ist, um das Verriegelungselement von der Ruhestellung in die Raststellung zu bringen.

Die korrekte Ausrichtung des Verriegelungselements in die Raststellung wird dadurch weiter verbessert, wenn die Schutzhülle ein zweites Arretiermittel aufweist, das mindestens um die Breite des Rasthakens vom ersten Arretiermittel beabstandet an der Innenseite der Schutzhülle befestigt ist und einen Anschlag für das Verriegelungselement bildet. Damit wird ein Überdrehen des Verriegelungselements vermieden und somit die sichere Arretierung der Schutzhülle in der Schließstellung verbessert.

Zur Vereinfachung der Herstellung können die Schutzhülle und das Basisteil ein einstückiges Spritzgussteil bilden. Weiter können die Schutzhülle und das Basisteil durch einen Falz verbunden sein, der ein Schwenkgelenk der Schutzhülle bildet. Auch diese Ausführungsform vereinfacht die Herstellung.

Die Erfindung wird nachfolgend anhand eines Ausführungsbeispiels unter Bezug auf die beigefügten schematischen Zeichnungen mit weiteren Einzelheiten erläutert. In diesen zeigen:
- Fig. 1: eine perspektivische Ansicht einer Sicherheitsvorrichtung nach einem erfindungsgemäßen Ausführungsbeispiel ohne Kanüle;
- Fig. 2: eine perspektivische Ansicht des Verriegelungselements;
- Fig. 3: einen Längsschnitt durch die Sicherheitsvorrichtung gemäß Fig. 1 mit einer Kanüle, wobei das Verriegelungselement sich in der Ruhestellung befindet;
- Fig. 4: die Sicherheitsvorrichtung gemäß Fig. 3, wobei die Schutzhülle in einer teilgeöffneten Stellung ist;
- Fig. 5: die Sicherheitsvorrichtung gemäß Fig. 3, wobei sich die Schutzhülle in der Offenstellung für den Gebrauch der Kanüle befindet und
- Fig. 6: die Sicherheitsvorrichtung gemäß Fig. 3, wobei sich die Schutzhülle in der arretierten Schließstellung nach Gebrauch der Kanüle befindet.

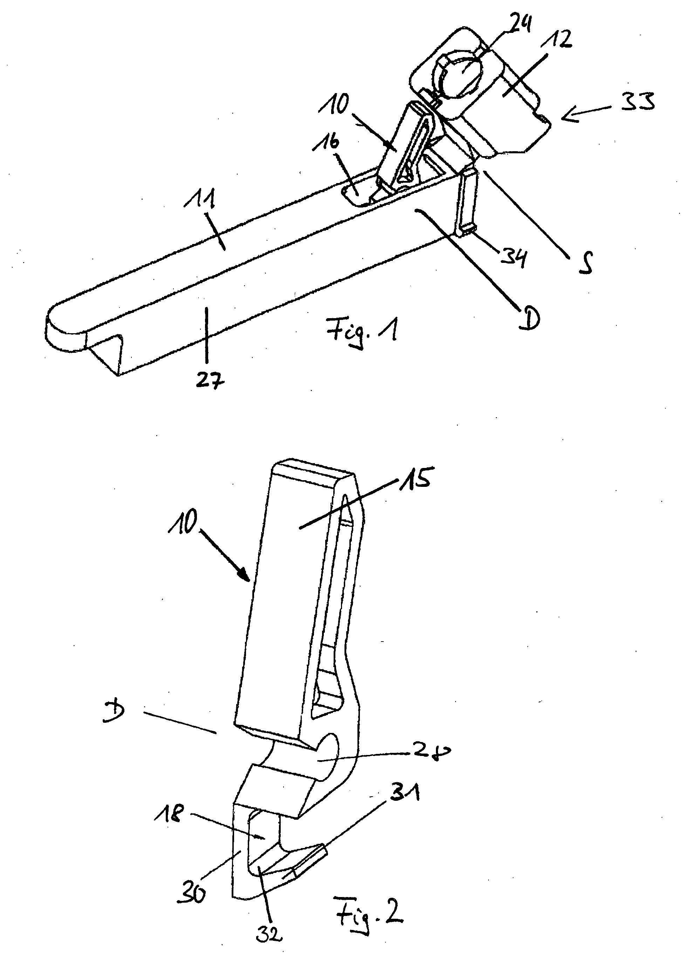

Fig. 1 zeigt eine Sicherheitsvorrichtung für eine Kanüle 5. Die Sicherheitsvorrichtung wird sowohl ohne Kanüle 5, wie in Fig. 1 dargestellt, als auch mit einer Kanüle 5 offenbart und beansprucht.

Die in Fig. 1 dargestellte Sicherheitsvorrichtung weist eine Schutzhülle 11 und ein Basisteil 12 auf, die miteinander verbunden sind. Das Basisteil 12 dient zur Positionierung der Kanüle 5 und bildet eine erste Öffnung 24 am proximalen Ende, d.h. an dem dem Benutzer zugewandten Ende des Basisteils 12. Eine zweite Öffnung 25, insbesondere kleinere Öffnung 25, des Basisteils 12 fluchtet mit der ersten Öffnung 24 derart, dass eine Hohlnadel der Kanüle 5 sich durch das Basisteil 12, insbesondere durch den sich zwischen den beiden Öffnungen 24, 25 befindlichen hohlen Innenraum 26 des Basisteils 12 hindurch erstrecken kann. Die Hohlnadel steht über das distale Ende des Basisteils 12 vor.

Die mit dem Basisteil 12 verbundene Schutzhülle 11 für die Kanüle 5 weist einen Hohlraum 13 auf, der sich in Längsrichtung der Kanüle 5 erstreckt. Der Hohlraum 13 wird seitlich von zwei Seitenwänden 27 und einer Wandung 17 begrenzt, die sich zwischen den beiden Seitenwänden 27 erstreckt und den Hohlraum 13 nach oben abschließt. Auf einer der oberen Wandung 17 gegenüberliegenden Seite ist eine Aufnahmeöffnung 14 ausgebildet, die etwas länger als die Kanüle 5 ist. Die Aufnahmeöffnung 14 bildet einen Schlitz, insbesondere Längsschlitz, oder einen Spalt, durch den die Kanüle 5 in den Hohlraum 13 bzw. die Kanülenkammer der Schutzhülle 11 hinein- bzw. herausbewegt werden kann.

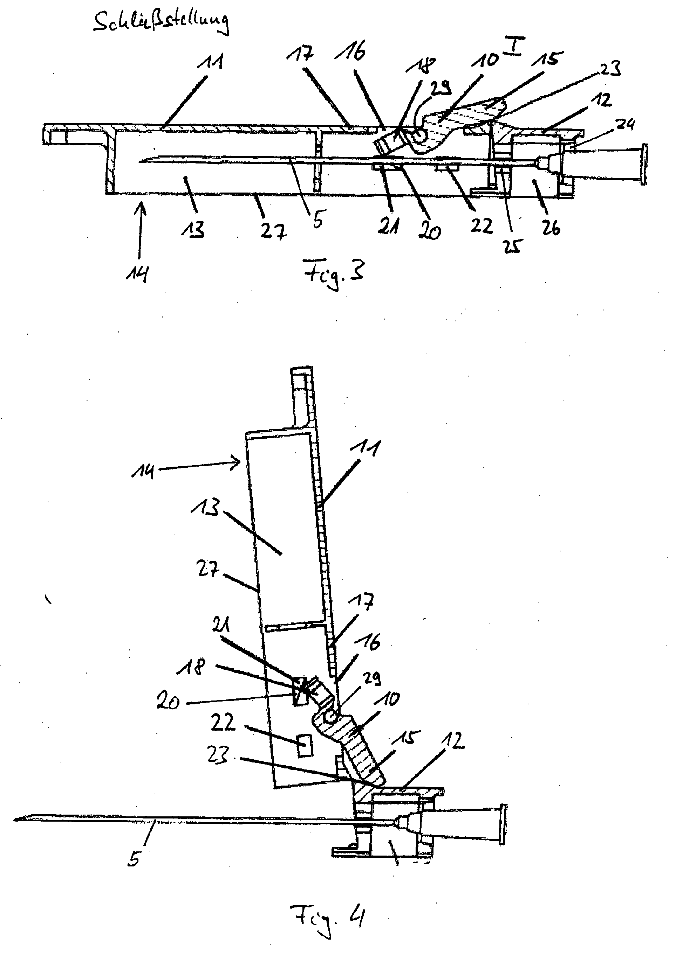

Bei dem Ausführungsbeispiel gemäß Fig. 3 erstrecken sich die Seitenwände 27 in der Schließstellung bis zum Basisteil 12. In der Schließstellung fluchtet der Hohlraum 13 mit den Öffnungen 24, 25 des Basisteils 12.

Zum Arretieren der Schutzhülle 11 in der in Fig. 3 dargestellten Schließstellung ist ein Schnappverschluss 33 vorgesehen. Der Schnappverschluss 33 umfasst jeweils an den Seitenwänden 27 der Schutzhülle 11 ausgeformte Vorsprünge 34, die in der Schließstellung der Schutzhülle 11 in entsprechende Schnappmulden (nicht dargestellt) des Basisteils 12 eingreifen. Die Vorsprünge 34 sind abgerundet, insbesondere hügelartig, geformt, so dass ein einfaches Lösen und Arretieren des Schnappverschlusses gewährleistet ist. Überdies sind die Seitenwände 27 elastisch nachgiebig bzw. auslenkbar, so dass die Seitenwände 27 zum Einschnappen der Vorsprünge 34 in die Schnappmulden nach innen, d.h. in Richtung zur Kanüle 5 bzw. zum Verriegelungselement 10, ausweichen können.

Das Verriegelungselement 10 ist an einem Lagerzapfen 29 drehbeweglich befestigt, der sich in einer Durchgangsöffnung 16 zwischen den beiden Seitenwänden 27 erstreckt. Der Lagerzapfen 29 ist vorzugsweise einstückig mit der Schutzhülle 11 ausgebildet, insbesondere durch Spritzgießen ausgeformt. Wie in Fig. 2 zu sehen ist, weist das Verriegelungselement 10 einen Kipphebel 15, der einen länglichen Griff bildet, sowie einen Rasthaken 18 auf. Der Kipphebel 15 und der Rasthaken 18 sind an entgegengesetzten Enden des Verriegelungselements 10 angeordnet. Zwischen dem Kipphebel 15 und dem Rasthaken 18 ist eine Lagerschale 28 ausgebildet, die sich quer zur Längsrichtung des Kipphebels 15 bzw. allgemein des Verriegelungselements 10 erstreckt. Die Lagerschale 28 weist einen Montageschlitz auf, durch den ein Lagerzapfen 29 der Schutzhülle 11 in die Lagerschale 28 eingepasst werden kann. Der Kipphebel 15, die Lagerschale 28 und der Rasthaken 18 bilden ein einstückiges Bauteil, insbesondere ein spritzgegossenes Bauteil.

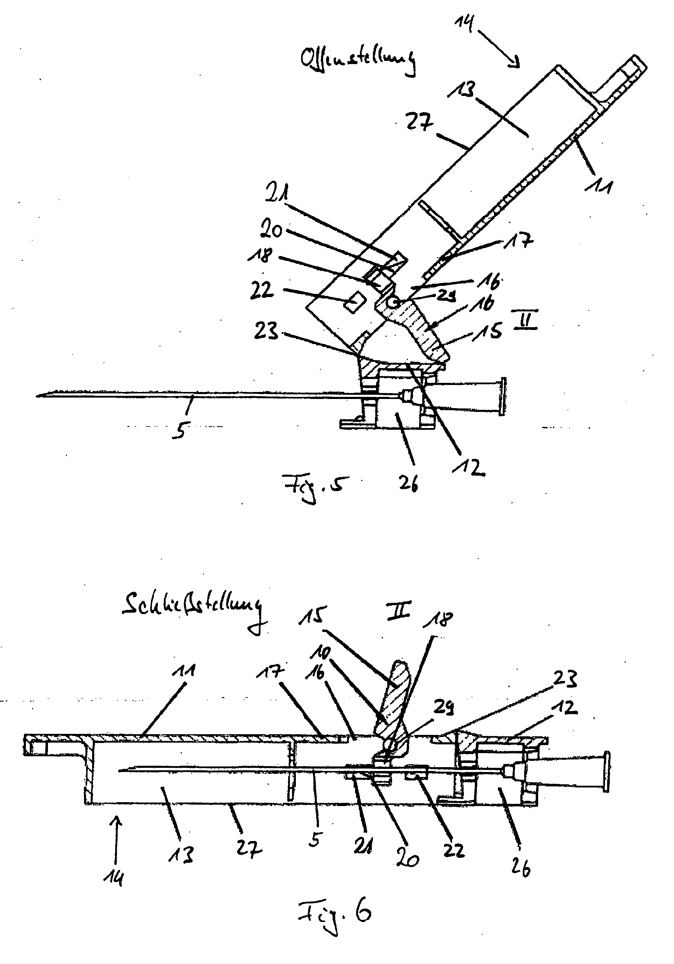

Der Rasthaken 18 erstreckt sich im Wesentlichen seitlich zum Verriegelungselement 10. Das bedeutet, dass sich der Rasthaken in einer Richtung parallel zur Drehachse D des Verriegelungselements 10 öffnet. Der Rasthaken 18 weist konkret eine Rückwand 30 und einen Haken 31 auf, der an der Unterkante der Rückwand 30 angeformt ist. Durch die Rückwand 30 ist der Haken 31 von der Lagerschale 28 beabstandet und bildet eine Aufnahmemulde 32 für die Kanüle 5. In de Schließstellung der Schutzhülle 11 und der Raststellung II des Verriegelungselements 10 ist die Aufnahmemulde 32 koaxial zur ersten Öffnung 24 des Basisteils 12 angeordnet (Fig. 6). Damit ist sichergestellt, dass die Kanüle 5 in der Schließstellung der Schutzhülle 11 und der Raststellung II des Verriegelungselements 10 in der Aufnahmemulde 32 angeordnet ist. Der Rasthaken 18 bzw. allgemein das Verriegelungselement 10 arretiert somit die Kanüle 5 in der Schutzhülle 11.

Der Lagerzapfen 29 ist mit den Seitenwänden 27 verbunden und befindet sich, wie in Fig. 3 dargestellt, nahe an der Wandung 17 bzw. nahe an der Oberkante der Schutzhülle 11, d.h. auf der von der Aufnahmeöffnung 14 der Schutzhülle 11 abgewandten Seite des Hohlraums 13. Allgemein ist der Lagerzapfen oberhalb einer imaginären Ebene angeordnet, die die Seitenwand 27 der Schutzhülle 11 in Längsrichtung der Schutzhülle 11 mittig schneidet. Außerdem ist der Lagerzapfen 29 im Bereich des proximalen Endes der Schutzhülle 11, also nahe am Basisteil 12 angeordnet. Im Bereich des Lagerzapfens 29 ist die obere Wandung 17 durchbrochen und bildet eine Durchgangsöffnung 16. Mit anderen Worten erstreckt sich der Lagerzapfen 29 zwischen den beiden Seitenwänden 27 der Schutzhülle 11 durch die Durchgangsöffnung 16. Der Lagerzapfen 29 ist vorzugsweise einstückig mit der Schutzhülle 11 ausgeformt.

Im montierten Zustand, wie in Fig. 3 dargestellt, ist das Verriegelungselement 10 drehbar mit der Schutzhülle 11 verbunden derart, dass das Verrieglungselement 10 bei einer Schwenkbewegung der Schutzhülle mitgenommen wird. Konkret ist das Verriegelungselement mit der Lagerschale 28 am Lagerzapfen 29 befestigt.

Der Kipphebel 15 ragt durch die Durchgangsöffnung 16 nach außen vor und ist somit zugänglich. Der Rasthaken 18 befindet sich im Hohlraum 13. Wie in Fig. 3 dargestellt, ist die Länge des Kipphebels 15 so bemessen, dass dieser auf dem angrenzenden Gehäuse aufliegt. Im geschlossenen Zustand gemäß Fig. 3 ist dies der proximale Rand der Schutzhülle 11, der die Durchgangsöffnung 16 begrenzt.

Der Lagerzapfen 29 ist so angeordnet, dass die Drehachse D des Verriegelungselements 10 parallel zur Schwenkachse S der Schutzhülle 11 verläuft. Dies ist in Fig. 1 dargestellt. Die Schwenkachse S ist durch einen Falz 23 gebildet, der ein Scharnier oder Schwenkgelenk zwischen dem Basisteil 12 und der Schutzhülle 11 bildet. Der Abstand zwischen der Drehachse D des Verriegelungselements 10 und der Schwenkachse S der Schutzhülle 11 ist kleiner als die Länge des Kipphebels 15, d.h. kleiner als der Abstand zwischen der Drehachse D und dem freien Ende des Kipphebels 15. Da die Drehachse D bzw. der Lagerzapfen 29 beim Verschwenken der Schutzhülle 11 sich auf einer Kreisbahn bewegt, deren Radius dem Abstand zwischen der Drehachse D und der Schwenkachse S entspricht, wird durch den längeren Kipphebel 15 erreicht, dass dieser bei der Schwenkbewegung der Schutzhülle 11 auf der Oberseite des Basisteils 12 abgleitet. Dazu ist das freie Ende des Kipphebels 15 abgerundet.

An einer der Seitenwände 27 der Schutzhülle 11 befindet sich ein erstes Arretiermittel 20. Der Abstand zwischen dem Arretiermittel 20 und der oberen Wandung 17 der Schutzhülle 11 ist so bemessen, dass das Verriegelungselement 10 in der in Fig. 3 dargestellten Ruhestellung I einerseits mit dem Rasthaken 18 am ersten Arretiermittel 20 und andererseits mit dem Kipphebel 15 am Gehäuse der Schutzhülle 11 bzw. des Basisteils 12 anliegt. Dadurch ist das Verriegelungselement 10 in der Ruhestellung I lösbar fixiert.

Das Arretiermittel 20 weist eine Gleitschräge 21 auf, die beispielsweise in den Fig. 4, 5 gut zu sehen ist. Die Gleitschräge 21 ist in der Drehebene des Verriegelungselements 10 angeordnet, so dass dieses beim Überführen von der Ruhestellung I in die Raststellung II auf der Gleitschräge 21 gleitet. Konkret weist die Gleitschräge 21 einen höchsten Punkt auf, der sich am proximalen Ende des Arretiermittels 20 befindet. Die Gleitschräge 21 bildet also einen Anstieg, der sich entlang der Seitenwand 27 ausgehend vom distalen Ende des Arretiermittels 20 zum proximalen Ende des Arretiermittels 20 erstreckt. Mit anderen Worten steigt die Gleitschräge 21 vom distalen Ende des Arretiermittels 20 zum proximalen Ende des Arretiermittels 20 an. Das Arretiermittel 20 weist eine steil abfallende, insbesondere senkrecht zur Seitenwand 27 angeordnete proximale Anschlagfläche auf, um das Verriegelungselement 10 in der Raststellung II zu halten.

In diesem Zusammenhang wird darauf hingewiesen, dass das Basisteil 12 am proximalen Ende und die Spitze der Schutzhülle 11 am distalen Ende der Sicherheitsvorrichtung angeordnet ist. Bauteile, die sich näher am Basisteil 12 befinden als ein Bezugsbauteil, sind also gegenüber dem Bezugsbauteil weiter proximal angeordnet. Umgekehrt sind Bauteile, die sich näher an der Spitze der Schutzhülle 11 befinden als ein Bezugsbauteil, gegenüber dem Bezugsbauteil weiter distal angeordnet.

Der Längsabstand des ersten Arretiermittels 20 vom proximalen Ende der Schutzhülle 11 ist so gewählt, dass der Rasthaken 18, insbesondere die Unterkante der Rückwand 30, in der Raststellung II (siehe Fig. 5) in einer Position im Wesentlichen parallel zur oberen Wandung 17 fixiert ist. Dazu liegt der Rasthaken 18 an einer proximalen Kante des ersten Arretiermittels 20 an.

In längsaxialer Richtung beabstandet vom ersten Arretiermittel 20 ist ein zweites Arretiermittel 22 an derselben Innenseite 27 der Schutzhülle 11 vorgesehen. In der Raststellung II gemäß Fig. 5 befindet sich der Rasthaken 18 zwischen dem ersten und zweiten Arretiermittel 20, 22, insbesondere zwischen einer proximalen Anschlagfläche des ersten Arretiermittels 20 und einer distalen Anschlagfläche des zweiten Arretiermittels 22.

Grundsätzlich können die Arretiermittel 20, 22 einstückig mit der Seitenwand 27 bzw. allgemein mit der Schutzhülle 11 ausgebildet, insbesondere als Verdickung der Seitenwand 27 ausgeformt, sein. Die Arretiermittel 20, 22 können auf der Wandstärke der Seitenwand 27 aufbauen oder dieselbe Wandstärke wie die Seitenwand 27 aufweisen. Im letzteren Fall bildet die Seitenwand 27 im Bereich der Arretiermittel 20, 22 auf einer Außenseite der Schutzhülle 11 eine Mulde, die der Tiefe der Arretiermittel 20, 22 entspricht. Die Arretiermittel 20, 22 können von der Außenseite der Schutzhülle 11 geprägt sein.

Die Sicherheitsvorrichtung funktioniert wie folgt:

Vor Gebrauch der Sicherheitsvorrichtung befindet sich die Schutzhülle 11 der in Fig. 3 dargestellten Schließstellung, wobei die Schutzhülle 11 nicht durch den Schnappverschluss mit dem Basisteil 12 verriegelt ist. Das Verriegelungselement 15 befindet sich in der in Fig. 3 dargestellten Ruhestellung I, in der der Rasthaken 18 oberhalb der Kanüle 5 angeordnet ist. Die Ruhestellung I ist durch die Anlage des Rasthakens 18 an der Oberseite des ersten Arretiermittels 20 oder an der in der Schutzhülle 11 angeordneten Kanüle 5 sichergestellt. Durch die lösbare Fixierung des Verriegelungselements 10 in der Ruhestellung I ist eine einfache Verbindung der Kanüle 5 mit der Sicherheitsvorrichtung bei der Montage möglich.

In Fig. 4 ist dargestellt, dass die Schutzhülle 11 in eine teilgeöffnete Stellung, also in eine Stellung zwischen der Schließstellung und der Offenstellung verschwenkt ist. Dabei wird das Verriegelungselement 10 mitbewegt, d.h. die Drehachse D wird auf einer Kreisbahn um den Mittelpunkt der Schwenkachse S bewegt. Dabei gelangt der Kipphebel 15, konkret das freie Ende des Kipphebels 15, in Anlage mit dem Basisteil 12. Ferner ist in Fig. 4 zu erkennen, dass durch die Schwenkbewegung der Schutzhülle 11 das Verriegelungselement 10 gedreht wird, so dass der Rasthaken 18 auf der Gleitschräge 21 gleitet bzw. an dieser entlanggeführt wird. Das hierfür erforderliche Drehmoment wird durch die Bewegung des Lagerzapfens 29 einerseits und die Anlage des freien Endes des Kipphebels 15 am Basisteil 12 andererseits erzeugt. Das Verriegelungselement 10 bewegt sich somit in einer zur Schwenkrichtung der Schutzhülle 11 entgegengesetzten Richtung. Aufgrund der Parallelität der Drehachse D und der Schwenkachse S erfolgen die Drehbewegungen in derselben Ebene.

Bei der Bewegung des Verriegelungselements 10 von der Ruhestellung I in die Raststellung II wird der Rasthaken 18 aufgrund der Gleitschräge 21 parallel zur Drehachse bzw. zum Lagerzapfen 29, d.h. aus der Zeichnungsebene heraus, ausgelenkt. Der Rasthaken 18 weist eine elastische Rückstellkraft auf, so dass der Rasthaken 18 nach Passieren des ersten Arretiermittels 20 in die Drehebene zurückspringt. Durch die steil abfallende, insbesondere senkrecht zur Seitenwand 27 angeordnete proximale Anschlagfläche des ersten Arretiermittels 20, wird verhindert, dass der Rasthaken 18 in entgegengesetzte Richtung über das erste Arretiermittel 20 zurück bewegbar ist. Ebenso wird eine weitere Bewegung des Verriegelungselements 10 nach Erreichen der Raststellung II durch die distale Anschlagfläche des zweiten Arretiermittels 22 verhindert.

In Fig. 5 ist die Offenstellung der Schutzhülle 11 dargestellt. In der Offenstellung ist die Kanüle 5 frei zugänglich und kann zum Injizieren bzw. Punktieren verwendet werden. Durch die Schwenkbewegung der Schutzhülle 11 und die damit erzeugte Relativbewegung des Verriegelungselements 15 bezogen auf die Schutzhülle 11 ist dieses an dem ersten Arretiermittel 20 vollständig vorbeibewegt und liegt am proximalen Ende des ersten Arretiermittels 20 an. Gleichzeitig hat das freie Ende des Kipphebels 15 eine translatorische Bewegung beschrieben und das proximale Ende des Basisteils 12 erreicht. In dieser Position befindet sich der Rasthaken 18, insbesondere die Unterkante der Rückwand 30, parallel zur Längsachse der Schutzhülle 11, und zwar konkret in einer Position, in der sich nach dem Schließen der Schutzhülle 11 die Kanüle 5 befindet. Der Rasthaken 18 befindet sich also in der Raststellung II, insbesondere in einer vorläufigen Raststellung, die nach dem Schließen der Schutzhülle 11 zum Arretieren derselben führt.

Fig. 6 zeigt die Sicherheitsvorrichtung in der arretierten Schließstellung nach dem Gebrauch der Kanüle 5. Das Verriegelungselement 15 befindet sich relativ zur Schutzhülle 11 in derselben Position wie in der komplett geöffneten Stellung gemäß Fig. 5. Der Kipphebel 15 ist in dieser Position in etwa senkrecht zur Wandung 17 der Schutzhülle 11 angeordnet. Durch die Bewegung der Schutzhülle 11 von der Offenstellung in die in Fig. 6 dargestellte Schließstellung wird der Rasthaken 18 mitgenommen und in Richtung Kanüle 5 bewegt. Der Rasthaken 18 trifft mit einer Unterseite auf die Kanüle 5 auf, wird durch den Widerstand der Kanüle 5 seitlich in Richtung der Seitenwand 27, die die Arretiermittel 20, 22 trägt, ausgelenkt und schnappt bei Erreichen der Schließstellung unterhalb der Kanüle 5 zurück. Durch die Auslenkung wird der Rasthaken 18 im Wesentlichen tiefer zwischen die beiden Arretiermittel 20, 22 gedrückt, so dass ein Ausweichen des Rasthakens 18 verhindert wird. Der Rasthaken 18 verrastet auf diese Weise mit der Kanüle 5 und arretiert diese damit in der Schutzhülle 11. Gleichzeitig wird der Schnappverschluss zwischen der Schutzhülle 11 und dem Basisteil 12 geschlossen. Während des gesamten Schließvorganges der Schutzhülle 11 befindet sich das Verriegelungselement 11 in der Raststellung II und wird mit der Kanüle 5 verriegelt, wenn die Schließstellung erreicht ist. Zur Unterstützung des Rastvorgangs mit der Kanüle 5 kann der Kipphebel 15 manuell gegriffen und in der Raststellung II gehalten werden. Der Anwender kann also mit einer Hand den Kipphebel 15 greifen und in der Raststellung II fixieren und gleichzeitig mit der anderen Hand das Basisteil 12, insbesondere mit der darin angeordneten Kanüle 5 greifen und um die Schwenkachse S verschwenken, bis die Kanüle 5 mit dem Rasthaken 18 verrastet bzw. in der Aufnahmemulde 32 des Rasthakens 18 angeordnet und die Schutzhülle 11 am Basisteil 12 eingeschnappt ist.

Zusammengefasst wird das Verriegelungselement 10 beim ersten Öffnen der Schutzhülle verdreht und gelangt von der Ruhestellung I in die Raststellung II, wenn die Schutzhülle 11 die Offenstellung erreicht. Dann ist das Verriegelungselement 10 in der Raststellung II fixiert und behält diese bei, wenn die Schutzhülle 11 nach dem Gebrauch der Kanüle 5 wieder verschlossen wird. Wenn die Schließstellung wieder erreicht ist, verrastet das Verriegelungselement 10 mit der Kanüle 5 und arretiert die Schutzhülle 11.

### Bezugszeichenliste

- 5: Kanüle
- 10: Verriegelungselement
- 11: Schutzhülle
- 12: Basisteil
- 13: Hohlraum
- 14: Aufnahmeöffnung
- 15: Kipphebel
- 16: Durchgangsöffnung
- 17: Wandung
- 18: Rasthaken
- 19: Innenseite
- 20: erstes Arretiermittel
- 21: Gleitschräge
- 22: zweites Arretiermittel
- 23: Falz
- 24: erste Öffnung
- 25: zweite Öffnung
- 26: Innenraum
- 27: Seitenwände
- 28: Lagerschale
- 29: Lagerzapfen
- 30: Rückwand
- 31: Haken
- 32: Aufnahmemulde
- 33: Schnappverschluss
- 34: Vorsprünge

## Patentansprüche

1. Sicherheitsvorrichtung für eine Kanüle (5) umfassend ein Verriegelungselement (10) und eine Schutzhülle (11), die mit einem Basisteil (12) verbunden ist und einen Hohlraum (13) mit einer Aufnahmeöffnung (14) für die Kanüle (5) aufweist, wobei die Schutzhülle (11) in eine Offenstellung und in eine Schließstellung um eine Schwenkachse S schwenkbar ist, wobei jeweils in der Schließstellung eine im Basisteil (12) positionierbare Kanüle (5) im Hohlraum (13) geschützt angeordnet und die Schutzhülle (11) durch das Verriegelungselement (10), das um eine parallel zur Schwenkachse (S) verlaufende Drehachse (D) relativ zur Schutzhülle (11) von einer Ruhestellung (I) in eine Raststellung (II) drehbar ist, arretierbar ist,
**dadurch gekennzeichnet, dass**
das Verriegelungselement (10) zur Erzeugung der Drehbewegung durch eine Schwenkbewegung der Schutzhülle (11) von der Schließstellung in die Offenstellung betätigbar ist.

2. Sicherheitsvorrichtung nach Anspruch 1,
**dadurch gekennzeichnet, dass**
das Verriegelungselement (10) einen Kipphebel (15) aufweist, der durch eine Durchgangsöffnung (16) nach außen ragt, die in einer Wandung (17) der Schutzhülle (11) gegenüber der Aufnahmeöffnung (14) angeordnet ist.

3. Sicherheitsvorrichtung nach Anspruch 2,
**dadurch gekennzeichnet, dass**
der Kipphebel (15) bei der Schwenkbewegung der Schutzhülle (11) von der Schließstellung in die Offenstellung am Basisteil (12) derart abstützbar ist, dass das Verriegelungselement (10) in die Raststellung II bewegbar ist.

4. Sicherheitsvorrichtung nach Anspruch 2 oder 3,
**dadurch gekennzeichnet, dass**
der Abstand zwischen der Drehachse D des Verriegelungselements (10) und der Schwenkachse S der Schutzhülle (11) kleiner als der Abstand zwischen der Drehachse D und dem freien Ende des Kipphebels (15) ist.

5. Sicherheitsvorrichtung nach einem der Ansprüche 2 bis 4,
**dadurch gekennzeichnet, dass**
der Kipphebel (15) am freien Ende eine abgerundete Form zum Gleiten auf dem Basisteil (12) aufweist.

6. Sicherheitsvorrichtung nach einem der vorhergehenden Ansprüche,
**dadurch gekennzeichnet, dass**
das Verriegelungselement (10) zum Arretieren der sich in der Schließstellung befindlichen Schutzhülle (11) in der Raststellung II mit der Kanüle (5) verrastbar ist.

7. Sicherheitsvorrichtung nach Anspruch 6,
**dadurch gekennzeichnet, dass**
das Verriegelungselement (10) einen Rasthaken (18) mit einer Aufnahmemulde (32) für die Kanüle (5) aufweist, wobei sich die Aufnahmemulde (32) in der Schließstellung der Schutzhülle (11) und der Raststellung des Verriegelungselements (10) koaxial zu einer ersten Öffnung (24) des Basisteils (12) erstreckt.

8. Sicherheitsvorrichtung nach einem der vorhergehenden Ansprüche,
**dadurch gekennzeichnet, dass**
die Schutzhülle (11) mindestens ein erstes Arretiermittel (20) aufweist, das an einer Innenseite (19) der Schutzhülle (11) befestigt oder ausgeformt ist, um das Verriegelungselement (10) in der Raststellung II zu fixieren.

9. Sicherheitsvorrichtung nach Anspruch 8,
**dadurch gekennzeichnet, dass**
das Verriegelungselement (10) in der Ruhestellung I durch das erste Arretiermittel (20) lösbar fixiert ist.

10. Sicherheitsvorrichtung nach Anspruch 8 oder 9,
**dadurch gekennzeichnet, dass**
das erste Arretiermittel (20) eine Gleitschräge (21) aufweist, die in proximale Richtung entlang der Innenwand (19) ansteigt und in einer Rotationsebene des Verriegelungselements (10) angeordnet ist derart, dass das Verriegelungselement (10) bei der Bewegung von der Ruhestellung I in die Raststellung II auf der Gleitschräge (21) gleitet.

11. Sicherheitsvorrichtung nach einem der Ansprüche 7 bis 10,
**dadurch gekennzeichnet, dass**
die Schutzhülle (11) ein zweites Arretiermittel (22) aufweist, das mindestens um die Breite des Rasthakens (18) vom ersten Arretiermittel (20) beabstandet an der Innenseite (19) der Schutzhülle (11) befestigt ist und einen Anschlag für das Verriegelungselement (10) bildet.

12. Sicherheitsvorrichtung nach einem der Ansprüche 1 bis 8,
**dadurch gekennzeichnet, dass**
die Schutzhülle (11) und das Basisteil (12) ein einstückiges Spritzgussteil bilden.

13. Sicherheitsvorrichtung nach Anspruch 12,
**dadurch gekennzeichnet, dass**
die Schutzhülle (11) und das Basisteil (12) durch einen Falz (23) verbunden sind, der ein Schwenkgelenk der Schutzhülle (11) bildet.

## Claims

1. Safety device for a cannula (5), comprising a locking element (10) and a protective cover (11) which is connected to a base part (12) and has a cavity (13) having a receiving opening (14) for the cannula (5), the protective cover (11) being pivotable about a pivot axis S into an open position and into a closed position, wherein in the closed position a cannula (5) positionable in the base part (12) is arranged so as to be protected in the cavity (13) and the protective cover (11) is arranged to be retained in position by the locking element (10) which is rotatable, relative to the protective cover (11), about a rotational axis (D) running parallel to the pivot axis (S) from a rest position (I) to an engaged position (II),
**characterised in that**
the locking element (10) is operable to generate the rotational movement by a pivoting movement of the protective cover (11) from the closed position to the open position.

2. Safety device according to claim 1,
**characterised in that**
the locking element (10) has a rocker arm (15) which projects outwards through an aperture (16) arranged in a wall (17) of the protective cover (11) opposite the receiving opening (14).

3. Safety device according to claim 2,
**characterised in that**,
during the pivoting movement of the protective cover (11) from the closed position to the open position, the rocker arm (15) is arranged to be supported on the base part (12) in such a way that the locking element (10) can be moved into the engaged position II.

4. Safety device according to claim 2 or 3,
**characterised in that**
the distance between the rotational axis D of the locking element (10) and the pivot axis S of the protective cover (11) is smaller than the distance between the rotational axis D and the free end of the rocker arm (15).

5. Safety device according to any one of claims 2 to 4,
**characterised in that**
the rocker arm (15) has at its free end a rounded shape for sliding on the base part (12).

6. Safety device according to any one of the preceding claims,
**characterised in that**
for retaining the protective cover (11) in the engaged position II when the protective cover is in the closed position, the locking element (10) is arranged to engage with the cannula (5).

7. Safety device according to claim 6,
**characterised in that**
the locking element (10) has an engaging hook (18) with a receiving hollow (32) for the cannula (5), which receiving hollow (32), when the protective cover (11) is in the closed position and the locking element (10) is in the engaged position, extends coaxially with a first opening (24) in the base part (12).

8. Safety device according to any one of the preceding claims,
**characterised in that**
the protective cover (11) has at least one first retaining means (20) which is attached to, or formed on, an inner side (19) of the protective cover (11) in order to fix the locking element (10) in the engaged position II.

9. Safety device according to claim 8,
**characterised in that**
the locking element (10) is releasably fixed in the rest position I by the first retaining means (20).

10. Safety device according to claim 8 or 9,
**characterised in that**
the first retaining means (20) has a slide slope (21) which ascends in the proximal direction along the inner wall (19) and is arranged in a plane of rotation of the locking element (10) in such a way that the locking element (10) slides on the slide slope (21) as it moves from the rest position I to the engaged position II.

11. Safety device according to any one of claims 7 to 10,
**characterised in that**
the protective cover (11) has a second retaining means (22) which is attached to the inner side (19) of the protective cover (11) so as to be spaced apart from the first retaining means (20) by at least the width of the engaging hook (18) and forms a stop for the locking element (10).

12. Safety device according to any one of claims 1 to 8,
**characterised in that**
the protective cover (11) and the base part (12) form a one-piece injection-moulded part.

13. Safety device according to claim 12,
**characterised in that**
the protective cover (11) and the base part (12) are connected by a hinge (23) which forms a pivot joint of the protective cover (11).

## Revendications

1. Dispositif de sécurité pour une canule (5) comprenant un élément de verrouillage (10) et une coque de protection (11) qui est reliée à une partie de base (12) et présente un espace creux (13) avec une ouverture de logement (14) pour la canule (5), étant entendu que la coque de protection (11) peut basculer autour d'un axe de basculement S dans une position ouverte et une position fermée, étant entendu que dans la position fermée, une canule (5) pouvant être positionnée dans la partie de base (12) est agencée de façon protégée dans l'espace creux (13) et la coque de protection (11) peut être bloquée au moyen de l'élément de verrouillage (10), qui peut pivoter par rapport à la coque de protection (11) d'une position de repos (I) à une position d'engagement (II) autour d'un axe de rotation (D) s'étendant parallèlement à l'axe de basculement (S),
**caractérisé**
**en ce que** l'élément de verrouillage (10) peut être actionné par un mouvement de basculement de la coque de protection (11) de la position fermée vers la position ouverte pour produire le mouvement de rotation.

2. Dispositif de sécurité selon la revendication 1,
**caractérisé**
**en ce que** l'élément de verrouillage (10) présente un levier basculant (15) qui dépasse à l'extérieur au travers d'une ouverture passante (16) qui est pratiquée dans une paroi (17) de la coque de protection (11) en opposition à l'ouverture de logement (14).

3. Dispositif de sécurité selon la revendication 2,
**caractérisé**
**en ce que** lors du mouvement de basculement de la coque de protection (11) de la position fermée vers la position ouverte, le levier basculant (15) peut prendre appui sur la partie de base (12) de telle sorte que l'élément de verrouillage (10) peut être déplacé dans la position d'engagement (II).

4. Dispositif de sécurité selon la revendication 2 ou 3,
**caractérisé**
**en ce que** la distance entre l'axe de rotation (D) de l'élément de verrouillage (10) et l'axe de basculement (S) de la coque de protection (11) est plus petite que la distance entre l'axe de rotation (D) et l'extrémité libre du levier basculant (15).

5. Dispositif de sécurité selon l'une des revendications 2 à 4,
**caractérisé**
**en ce que** le levier basculant (15) présente à son extrémité libre une forme arrondie pour glisser sur la partie de base (12).

6. Dispositif de sécurité selon l'une des revendications précédentes,
**caractérisé**
**en ce que** l'élément de verrouillage (10) peut être immobilisé dans la position d'engagement (II) avec la canule (5) afin de bloquer la coque de protection (11) se trouvant dans la position fermée.

7. Dispositif de sécurité selon la revendication 6,
**caractérisé**
**en ce que** l'élément de verrouillage (10) présente un crochet d'engagement (18) avec une gouttière de logement (32) pour la canule (5), étant entendu que dans la position fermée de la coque de protection (11) et dans la position d'engagement de l'élément de verrouillage (10), la gouttière de logement (32) s'étend de façon coaxiale avec une première ouverture (24) de la partie de base (12).

8. Dispositif de sécurité selon l'une des revendications précédentes,
**caractérisé**
**en ce que** la coque de protection (11) présente au moins un premier moyen de blocage (20) qui est fixé ou formé sur un côté intérieur (19) de la coque de protection (11) afin de fixer l'élément de verrouillage (10) dans la position d'engagement (II).

9. Dispositif de sécurité selon la revendication 8,
**caractérisé**
**en ce que** l'élément de verrouillage (10) est fixé de façon amovible dans la position de repos (I) par le premier moyen de blocage (20).

10. Dispositif de sécurité selon la revendication 8 ou 9,
**caractérisé**
**en ce que** le premier moyen de blocage (20) présente un biseau de glissement (21) qui va en montant dans la direction proximale le long de la paroi intérieure (19) et qui est agencé dans un plan de rotation de l'élément de verrouillage (10) de telle sorte que lors du mouvement de la position de repos (I) vers la position d'engagement (II), l'élément de verrouillage (10) glisse sur le biseau de glissement (21).

11. Dispositif de sécurité selon l'une des revendications 7 à 10,
**caractérisé**
**en ce que** la coque de protection (11) présente un deuxième moyen de blocage (22) qui est fixé à une distance d'au moins la largeur du crochet d'engagement (18) du premier moyen d'arrêt (20) sur le côté intérieur (19) de la coque de protection (11) et qui forme une butée pour l'élément de verrouillage (10).

12. Dispositif de sécurité selon l'une des revendications 1 à 8,
**caractérisé**
**en ce que** la coque de protection (11) et la partie de base (12) forment une pièce moulée par injection d'une seule pièce.

13. Dispositif de sécurité selon la revendication 12,
**caractérisé**
**en ce que** la coque de protection (11) et la partie de base (12) sont reliées par une feuillure (23) qui forme une charnière de basculement de la coque de protection (11).
